(19) Europäisches Patentamt
European Patent Office
Office européen des brevets

(11) **EP 2 353 619 A1**

(12) **EUROPEAN PATENT APPLICATION**

(43) Date of publication:
**10.08.2011 Bulletin 2011/32**

(51) Int Cl.:
*A61L 24/00* (2006.01)   *A61L 24/02* (2006.01)
*A61L 27/02* (2006.01)   *A61L 27/12* (2006.01)
*A61L 27/54* (2006.01)

(21) Application number: **10153044.2**

(22) Date of filing: **09.02.2010**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(71) Applicant: **Bone Support AB
223 70 Lund (SE)**

(72) Inventors:
• **Karlsson, Veronica
S-237 32, Bjärred (SE)**

• **Nilsson, Malin
S-824 43, Hudiksvall (SE)**
• **Lidén, Eva
S-224 71, Lund (SE)**

(74) Representative: **Nilausen, Kim
Zacco Denmark A/S
Hans Bekkevolds Allé 7
2900 Hellerup (DK)**

(54) **Preparation of Bone Cement Compositions**

(57)    A method for the preparation of injectable ready-to-use paste bone cement compositions by mixing a dry inorganic bone cement powder comprising a particulate calcium sulfate hemihydrate capable of hardening *in vivo* by hydration of the calcium sulfate hemihydrate forming calcium sulfate dihydrate, an aqueous liquid and an additive that normally retards the setting process, said method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component

b) mixing the bone cement powder with the aqueous liquid for a period of time

c) allowing the calcium sulfate hemihydrate to dissolve at least partially and the formation of calcium sulfate dihydrate crystallization nuclei, and

d) admixing the additive

surprisingly shortens the setting times for the cement comprising the additive that retard the setting process to the level observed in the absence of the additive and enables a complete hydration of calcium sulfate hemihydrate to calcium sulfate dihydrate.

EP 2 353 619 A1

**Description**

**CROSS-REFERENCE TO RELATED APPLICATIONS**

Not relevant

**BACKGROUND OF THE INVENTION**

**1. Field of the Invention**

The present invention relates to the preparation of an injectable paste from powdery calcium sulfate based bone cement compositions and a medicament, which compositions are made ready to use, by mixing with an aqueous liquid.

**Introduction**

The life expectancy of the world population has increased tremendously during the last 50 years, and according to the forecasts there will be more people over 60 years of age than less than twenty years of age in Europe.

More people will need medical help for diseases related to age, which will increase the pressure of the hospitals.

Bone is the second most common material to be transplanted after blood. The most reliable method to repair bone defects is to use autogenous bone, i.e. bone taken from another site in the body. However, problems may occur at the second surgical site from where the graft is taken. To avoid these extra trauma allografts can be used i. e. bone graft between individuals of the same species. Allografts have a lower osteogenic capacity than autografts and the rate of new bone formation might be lower. They also have a higher resorption rate, a larger immunogenic response and less revascularisation of the recipient. Allografts must also be controlled for viruses since they can transfer, for example, HIV and hepatitis. The use of allografts is now the most common method for bone transplantation and repairing of bone defects. To solve the problems of supply, unpredictable strength and risk of infection, synthetic bone substitutes have become a realistic alternative. Thus, the demand for and use of synthetic bone substitutes is increasing rapidly.

Calcium sulfate hemihydrate, $CaSO-1/2H_2O$, CSH, was one of the first materials investigated as a substitute for bone grafts. Calcium sulfate hemihydrate implanted in areas of subcortical bone produces no further untoward reaction in the tissue than normally is present in a fracture. The new bone growing into calcium sulfate is normal bone, and no side effects attributable to the implantation of calcium sulfate hemihydrate have been noted in adjacent tissues or in distant organs.

The most important advantage with calcium sulfate is its excellent biocompatibility. The drawbacks are the rapid resorption and low strength, which makes it less useful in larger or non-contained defects and when the fracture healing exceeds 4-6 weeks.

When calcium sulfate hemihydrate is mixed with water, it will hydrate to calcium sulfate dihydrate, CSD, according to the below reaction scheme (1):

$$CaSO_4 \cdot 0.5\ H_2O + 1.5\ H_2O => CaSO_4 \cdot 2\ H_2O + Heat \quad (1)$$

The hydration reaction of calcium sulfate hemihydrate can be summarized in three phases.

1) The induction period starting immediately after the calcium sulfate hemihydrate powder is mixed with water. The calcium sulfate hemihydrate then dissolves and the solution becomes supersaturated with respect to calcium and sulfate ions. This leads to precipitation of the less soluble calcium sulfate dihydrate, CSD. In order for the hydration reaction to be able to proceed, these CSD nuclei have to have a radius that is larger than a "critical radius" (determined for each specific system). The induction period is critical for the hydration reaction and any disturbances in the solubility of calcium sulfate hemihydrate or growth of CSD-crystals in this phase will delay the further hydration reaction to a higher degree than occurrence of the same disturbance in later phase of the process.

2) The acceleratory or growth period starts when a sufficient number of CSD crystals have reached the critical size for them to act as nucleating embryos. The CSD nucleus formed will then grow and form large crystals. The crystals will eventually be sufficiently large to interlock with each other and the friction between crystals contributes to the strength of the formed material.

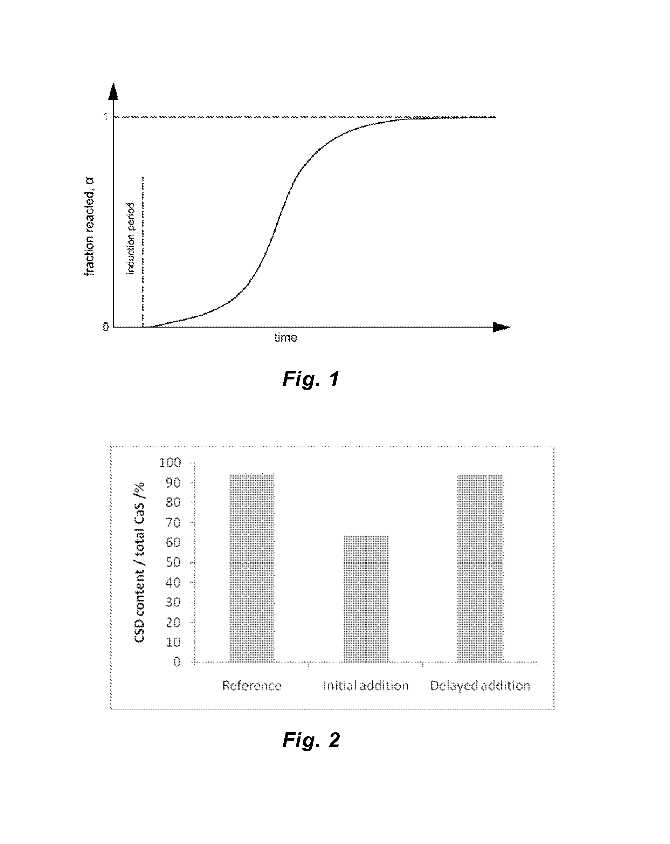

3) The third phase is relatively slow and consists of the completion of the hydration of the calcium sulfate hemihydrate as stated in N. B. Singh and B. Middendorf, Calcium sulphate hemihydrate hydration leading to gypsum crystallization, Progress in Crystal Growth and Characterization of Materials 53 (2007) 57-77 and as illustrated in figure 1 in the form of a schematic view showing the fraction of hydrated calcium sulfate dihydrate as a function of time.

For a variety of applications, it is desired to be able to mix different additives to calcium sulfate based bone cements. Bone substitutes comprising an antibiotic content are often requested to prevent or treat bone infections (Steven Gitelis and Gregory T. Brebach, The treatment of chronic osteomyelitis with a biodegradable antibiotic implant, Journal of Orthopaedic Surgery 2002 10(1): 53-60).

However, it has been found that addition of various additives such as antibiotics to bone cement compositions based on calcium sulfate hemihydrate interferes with or even prevents the hardening process.

Thus it has been found (Steven Gitelis and Gregory T. Brebach, *ibid)* that addition of antibiotics may retard or accelerate the hydration of calcium sulfate hemihydrate into calcium sulfate dihydrate significantly, or even prevent completion of the hydration and consequently, the hydraulic cement may no longer be suitable for use in clinical applications since its properties are significantly changed by the addition of the antibiotic. The incomplete/slow CSH hydration affects different properties of the final material and excludes a number of applications for the material

## 2. Description of the Related Art

Various bone cement compositions comprising ceramics hardening *in vivo* upon contact with water or body fluids and comprising a medicament have been disclosed.

WO 2004/078223 (Lidgren) discloses a bone mineral substitute material comprising a calcium phosphate component and hardened calcium sulphate and an additive such as an antioxidant, a vitamin, a hormone, an antibiotic, a cytostatic, a bisphosphonate, a growth factor, or a protein. The additive can be included in the particulate hardened calcium sulfate during its preparation or included in the sterile aqueous liquid of the composition, and in the experiments a slow release of iohexol or gentamycin was found when the additive was included in the particulate hardened calcium sulfate during its preparation.

In N. B. Singh and B. Middendorf, Calcium sulphate hemihydrate hydration leading to gypsum crystallization, Progress in Crystal Growth and Characterization of Materials 53 (2007) 57-77 it is disclosed that the presence of e.g. carboxylic acids retards the growth of gypsum crystals during hemihydrate hydration.

Richelsoph et al, Elution Behavior of Daptomycin-loaded Calcium Sulfate Pellets, CLINICAL ORTHOPAEDICS AND RELATED RESEARCH, Number 461, pp 68-73, found that antibiotics often tend to disturb the setting of calcium sulphate hemihydrate and that when daptomycin incorporation of traditional accelerant techniques (addition of a small percentage of calcium sulphate dihydrate, addition of saline, increasing the temperature and decreasing the water content) all failed to improve the setting characteristics. Furthermore, Richelsoph found that when using potassium sulfate as accelerator a suitable pellet was produced and describes a two-step method in which calcium sulfate hemihydrate powder and a potassium sulfate solution were stirred vigorously for two minutes and allowed to rest for additionally one minute before daptomycin was added.

The hydration of calcium sulfate hemihydrate, CSH, into calcium sulfate dihydrate, CSD, may be retarded or prevented from being completed. This may lead to the result that the hydraulic cement will no longer be suitable for use in clinical applications since its properties are significantly changed by addition of an antibiotic. The incomplete/slow CSH hydration affects different properties of the final material and excludes the use of such a material for a number of applications. Accelerated hydration has also been reported when adding antibiotics, which leads to similar limitations.

It is an object of the invention to provide an inorganic bone mineral substitute composition comprising an additive having a desired effect, said additive having a retarding effect on the hardening of the inorganic bone mineral substitute composition when admixed together with an aqueous liquid for hardening of the cement composition without sacrificing the rate of setting, the completion of the hydration of CSH into CSD or the properties of the final product (after 30 minutes).

## SUMMARY OF THE INVENTION

The present invention relates in a first aspect to a method for the preparation of ready-to-use injectable bone cement paste compositions by mixing a dry inorganic bone cement powder comprising a particulate calcium sulfate hemihydrate capable of hardening *in vivo* by hydration of the calcium sulfate hemihydrate forming calcium sulfate dihydrate, an aqueous liquid and an additive, said method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time,

c) allowing the calcium sulfate hemihydrate to dissolve at least partially and the formation of calcium sulfate dihydrate crystallization nuclei, and

d) admixing the additive.

In a second aspect the invention relates to a method of prophylactic or therapeutic treatment of a disorder related to supportive tissues in a human or non-human animal subject, which method comprises providing to said subject a composition for an injectable inorganic bone mineral substitute paste material with the capability of being hardened in a body fluid *in vivo* by hydration of calcium sulfate hemihydrate forming calcium sulfate dihydrate, said composition comprising a dry inorganic bone cement powder composition comprising particulate calcium sulfate hemihydrate, an aqueous liquid and at least one bioactive agent active against said disorder, said method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time,
c) allowing the calcium sulfate hemihydrate to dissolve at least partially and the formation of calcium sulfate dihydrate crystallization nuclei, and
d) admixing the additive and
e) introducing the resulting inorganic bone mineral substitute material into said tissue.

In a third aspect the invention relates to a method of implanting a hardened inorganic bone mineral substitute in the form of hardened pellets, small beads, rods, or blocks to a supportive tissue in a human or non-human animal subject, said hardened inorganic bone mineral substitute being prepared by a method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time,
c) allowing the calcium sulfate hemihydrate to dissolve at least partially and the formation of calcium sulfate dihydrate crystallization nuclei, and
d) admixing the additive and
e) introducing the resulting inorganic bone mineral substitute material into said tissue.

In a fourth aspect the invention relates to a method of concomitant implanting an inorganic bone mineral substitute to a supportive tissue in a human or non-human animal subject and prophylactic or therapeutic administration of an antibiotic agent, wherein a hardened inorganic bone mineral substitute material is introduced into said tissue, said hardened inorganic bone mineral substitute material being prepared by a method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time,
c) allowing the calcium sulfate hemihydrate to dissolve at least partially and the formation of calcium sulfate dihydrate crystallization nuclei, and
d) admixing the antibiotic agent,
e) forming the resulting material into pellets, small beads, rods or blocks and allowing these to harden *ex vivo* and
f) introducing the resulting hardened inorganic bone mineral substitute material into said tissue.

In a fifth aspect the invention relates to an injectable paste composition comprising a particulate calcium sulfate hemihydrate capable of hardening *in vivo* by hydration of the calcium sulfate hemihydrate forming calcium sulfate dihydrate, an aqueous liquid and a bioactive agent, prepared by

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time
c) allowing the calcium sulfate hemihydrate to dissolve at least partially and the formation of calcium sulfate dihydrate crystallization nuclei, and

d) admixing the bioactive agent,

for use as a medicament for prophylactic or therapeutic treatment of a disorder related to supportive tissues in a human or non-human animal subject, which method comprises local administration to said subject, preferably by injection, of said composition comprising a prophylactic or therapeutic amount of said at least one bioactive agent, which is released from said composition, optionally while systemically and/or concomitantly administrating a prophylactic or therapeutic amount of at least one bioactive agent.

**Brief Description of the Drawings**

The invention is disclosed more in detail with reference to the drawings in which

Fig. 1 Shows a schematic view of the fraction of hydrated CSH as a function of time,
Fig. 2 shows to what extent the CSH was converted to CSD in three samples, and
Fig. 3 shows a photograph illustrating injection test

**Detailed Description of the Present Invention**

The present invention relates to a method for the preparation of ready-to-use injectable bone cement paste compositions by mixing a dry inorganic bone cement powder comprising a particulate calcium sulfate hemihydrate capable of hardening *in vivo* by hydration of the calcium sulfate hemihydrate forming calcium sulfate dihydrate, an aqueous liquid and an additive, said method comprising

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time,
c) allowing the calcium sulfate hemihydrate to dissolve at least partially and formation of calcium sulfate dihydrate crystallization nuclei, and
d) admixing the additive.

The resulting material may be introduced to a supportive tissue in a human or non-human animal subject directly by injection and allowed to harden *in vivo* or it may shaped into a desired shape such as pellets, small beads, rods, or blocks and left for hardening *ex vivo* and then be introduced to the supportive tissue. In the alternative the resulting paste material may be manually moulded and introduced into the supportive tissue and shaped in place and then allowed to harden *in vivo.*

Supportive tissue may be any part of the tissue supporting the body or functional parts thereof such as bone tissue.

In the present context the expressions "harden" and hardened" are used to designate setting reaction taking place when hydraulic cements react with water.

It has been found that the method used to mix calcium sulfate based bone substitutes with an additive is crucial for the properties of the materials. This is especially relevant when adding certain additives that retard the setting process. It has now surprisingly been found that the time of addition of the additive in the preparation of CSH-based bone cement has a significant effect on the setting process.

It has now surprisingly been found that when pre-mixing a hydraulic cement powder comprising powdered CSH and CSD as an accelerator and a powdered calcium phosphate component with the aqueous liquid to be used for preparing the cement and allowing the hydration reaction of the CSH start before adding the additive, it is possible to shorten the setting times for the cement comprising the additive to the level observed in the absence of the additive and to obtain a complete hydration of CSH to CSD without having to substitute usually preferred accelerators and without having to mix under vigorous stirring which would require the use of separate mixing equipment in the sterile operating rooms. The mixing may be carried out manually in a mixing tool which then also can serve as injection syringe.

The time needed for the hydration reaction to start depends on the length of the induction period for the particular system and it easily determined for each system by the skilled in the art by routine experiments. An initial mixing time of 25 seconds to one minute, especially about 30 seconds has been found suitable when carrying out the present invention. After the mixing, the material is suitably left for at least 2 minutes before addition of additive.

These time windows also leaves a sufficiently broad time span for using the resulting paste to absorb many unforeseen incidents or minor delays often occurring during surgery.

The calcium sulfate hemihydrate may be a or $\beta$-calcium sulfate hemihydrate, $\alpha$-calcium sulfate hemihydrate

being preferred, and suitably the powdered calcium sulfate hemihydrate has a particle size of less than 500 $\mu$m, preferable less than 100 $\mu$m and preferable 99% of the particles have a particle size less than 80 $\mu$m.

The particle size of the powdered calcium sulfate dihydrate accelerator suitably is less than 500 $\mu$m, preferably less than 150 $\mu$m, and most preferable less than 100 $\mu$m.

The particulate calcium sulfate dihydrate should be present in an amount between 0.1 and 10 wt%, preferably between 0.1 and 2 wt% of the calcium sulfate hemihydrate which is to react with the aqueous liquid

The powdered calcium phosphate component may e.g. be amorphous calcium phosphate (ACP), , monocalcium phosphate monohydrate (MCPM; $Ca(H_2PO_4) \cdot 2H_2O$), dicalcium phosphate dihydrate DCPD (brushite; $CaHPO_4 \cdot 2H_2O$), octacalcium phosphate ($Ca_8(HPO_4)_2(PO_4)_4 \cdot 5H_2O$), calcium deficient hydroxylapatite (CDHA; $Cag(HPO_4)(PO_4)_5(OH)$), tricalcium phosphate (TCP;

$Ca_3(PO_4)_2$), and hydroxylapatite (HA; $Ca_{10}(PO_4)_6(OH)_2$.

It is preferred that the powdered calcium phosphate component is hydroxylapatite or tricalcium phosphate, preferably hydroxylapatite or $\alpha$-tricalcium phosphate having a particle size less than 100 $\mu$m.

When the powdered calcium phosphate component is tricalcium phosphate it is suitable to add an accelerator known per se such as hardened particulate calcium phosphate or disodium hydrogen phosphate as an accelerator for the calcium phosphate component. The hardened calcium phosphate should have a particle size which is less than 100 $\mu$m, suitably less than 50 $\mu$m, and comprise between 0.1 and 10 wt%, preferably between 0.5 and 5 wt% of the calcium phosphate which is to react with an aqueous liquid.

The reaction of calcium phosphate to a calcium phosphate cement can also be accelerated by addition of a phosphate salt, for example disodium hydrogen phosphate ($Na_2HPO_4$), which may be added as dry particles or dissolved in the aqueous liquid. In this case, the accelerator should be present in the aqueous liquid at concentrations of 0.1-10 wt%, preferably 1-5 wt%.

In order to confer an initial strength to an inorganic bone mineral substitute material the calcium sulphate hemihydrate should comprise 2-80 wt%, preferably 10-30 wt% of the dry powder to be mixed with an aqueous liquid, when a calcium phosphate to be hardened is used. Likewise, the calcium phosphate to be hardened to a calcium phosphate cement should comprise 20-98 wt%, preferably 70-90 wt% of the dry powder. When using hydroxylapatite as the calcium phosphate component, the hydroxylapatite suitably comprises from 30 to 50 wt% of the dry powder, preferably about 40 wt%, in which case the CSH +CSD will constitute from 50 to 70 wt% of the dry powder, preferably about 60 wt%. In the composition, the aqueous liquid should comprise between 0.1 and 2 ml, preferably between 0.2 and 0.7 ml per gram powder.

An inorganic bone cement powder which may suitably be used in the method of the present invention is commercially available from BONESUPPORT AB, Lund, Sweden under the trade name CERAMENT™ BONE VOID FILLER.

In a preferred embodiment of the invention the additive is in the form of a bioactive agent.

An additive in the form of a bioactive agent may e.g. be an antioxidant, a vitamin, a hormone, an antibiotic, a cytostatic, a bisphosphonate, a growth factor, or a protein or peptide. The additive may also be a bone marrow aspirate or demineralised bone. Advantageously, the additive is a substance that induces, stimulates and/or accelerates bone formation, such as osteoinductive compounds and/or compounds reducing bone turn over.

Suitable bone inducing substances, which stimulate and/or accelerate bone formation, are growth factors and hormones. Growth factors and derivatives thereof are preferred, which are locally acting.

It is preferred to use growth factors, which are autologous and effective in connection with bone, tendon or cartilage. Such growth factors are for example transforming growth factor (TGF $\beta$3), bone morphogenic protein (BMP-2 or BMP-7), PTHrP, osteoprotegrin (OPG), Indian Hedgehog, RANKL, basic fibroblast, insulin-like growth factor (IgF-1 or IGF-2), platelet derived growth factors, and vascular growth factors. These endogenously produced growth factors may be used as an additive either as single entities or combined in a growth factor mixture in order to accelerate bone growth. Thus, it is preferred that an endogenously produced bioactive molecule is used as a substance that induces bone formation.

Examples of other bone stimulating compounds are parathyorid hormones and derivatives thereof, estrogens, progesterones, androgens, testosterones, calcitonin, somatomedin, and oxytocin, preferably also autologous, but they can also be produced according to procedures known within the art.

The enamel matrix proteins amelin-1, amelin-2 and ameloblastin can also be included as an additive. Such compounds may either be autologous or extracted from or produced by tissues or cells from other species, or synthetically produced or produced by other living cells or organisms.

Likewise, the cholesterol-lowering compound statin can also be included in order to induce, stimulate and/or accelerate bone formation.

Examples of suitable bone breakdown inhibitors are biphosphonates, osteocalcin, osteonectin and derivatives thereof, which can be included as an additive in the particulate calcium sulfate of the inventive composition and of the resulting substitute.

The bioactive agent can also be an anti-infectious substance, i. e. a compound with a static or cidal effect against invading foreign living material.

Such compounds include natural antibiotics as well as other semisynthetic and synthetic antibacterial or bacteriostatic compounds, which are acting against pathogenic and/or infectious microorganisms, e. g. staphylococci. Examples of antibiotics for bone infections are tetracycline-HCl, vancomycin, tobramycin, gentamycin, and cephalosporin.

Cytostatic agents, such as cis-platinum, ifosfamide, methotrexate, doxorubicin-HCl, arsenic trioxide, and retinoids or derivatives thereof can also be used as a bioactive agent. The bioactive agent can in a similar way be an antiviral compound, an antifungal compound, a tuberculostatic or tuberculocidal compound or an antiparasite compound.

In a preferred embodiment the bioactive agent is an antibiotic agent.

In the method of the invention further additives known per se for controlling the rheological properties, e.g. as described in applicant's WO 01/76649, may also be added and such addition is also considered an aspect of the present invention.

It is also considered an aspect of the present invention to add an X-ray contrast agent known per se, preferably a non-ionic water soluble contrast agent as described in applicant's WO 03/053488.

The invention further relates to a method of prophylactic or therapeutic treatment of a disorder related to supportive tissues in a human or non-human animal subject, which method comprises providing to said subject an injectable paste composition for an inorganic bone mineral substitute material with the capability of being hardened in a body fluid *in vivo* by hydration of calcium sulfate hemihydrate forming calcium sulfate dihydrate, said composition comprising a dry inorganic bone cement powder composition comprising particulate calcium sulfate hemihydrate, an aqueous liquid and at least one bioactive agent active against said disorder, said method comprising

   a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
   b) mixing the bone cement powder with the aqueous liquid for a period of time,
   c) allowing the calcium sulfate hemihydrate to dissolve at least partially and the formation of calcium sulfate dihydrate crystallization nuclei, and
   d) admixing the additive and
   e) introducing the resulting inorganic bone mineral substitute material into said tissue.

The disclosure concerning suitable and preferred embodiments stated above in connection with the explanation of the method for the preparation of a ready-to-use bone cement composition applies mutatis mutandum for this aspect of the invention.

Furthermore the invention relates to a method of implanting a hardened inorganic bone mineral substitute in the form of hardened pellets, small beads, rods, or blocks to a supportive tissue in a human or non-human animal subject, said hardened inorganic bone mineral substitute being prepared by a method comprising

   a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
   b) mixing the bone cement powder with the aqueous liquid for a period of time,
   c) allowing the calcium sulfate hemihydrate to dissolve at least partially and the formation of calcium sulfate dihydrate crystallization nuclei, and
   d) admixing the additive and
   e) introducing the resulting inorganic bone mineral substitute material into said tissue.

The mixture can be introduced to a bone defect by injection or moulded and inserted manually.

The disclosure concerning suitable and preferred embodiments stated above in connection with the explanation of the method for the preparation of a ready-to-use bone cement composition applies mutatis mutandum for this aspect of the invention.

Still further the invention relates to a method of concomitant implanting an inorganic bone mineral substitute to a supportive tissue in a human or non-human animal subject and prophylactic or therapeutic administration of an antibiotic agent, wherein a hardened inorganic bone mineral substitute material is introduced into said tissue, said hardened inorganic bone mineral substitute being prepared by a method comprising

   a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component

b) mixing the bone cement powder with the aqueous liquid for a period of time,
c) allowing the calcium sulfate hemihydrate to dissolve at least partially and the formation of calcium sulfate dihydrate crystallization nuclei, and
d) admixing the antibiotic agent,
e) forming the resulting material into pellets, small beads, rods, or blocks and allowing these to harden *ex vivo* and
f) introducing the resulting hardened inorganic bone mineral substitute material into said tissue.

The hardened material may be provided threaded on a string of a non-bioresorbable or bioresorbable material such as polylactide or polyglycol.

The disclosure concerning suitable and preferred embodiments stated above in connection with the explanation of the method for the preparation of a ready-to-use bone cement composition applies mutatis mutandum for this aspect of the invention.

Yet further the invention relates to an injectable paste composition comprising a particulate calcium sulfate hemihydrate capable of hardening *in vivo* by hydration of the calcium sulfate hemihydrate forming calcium sulfate dihydrate, an aqueous liquid and a bioactive agent, prepared by

a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
b) mixing the bone cement powder with the aqueous liquid for a period of time
c) allowing the calcium sulfate hemihydrate to dissolve at least partially and the formation of calcium sulfate dihydrate crystallization nuclei, and
d) admixing the bioactive agent,

for use as a medicament for prophylactic or therapeutic treatment of a disorder related to supportive tissues in a human or non-human animal subject, which method comprises local administration to said subject, preferably by injection, of said composition comprising a prophylactic or therapeutic amount of said at least one bioactive agent, which is released from said composition, optionally while systemically and/or concomitantly administrating a prophylactic or therapeutic amount of at least one bioactive agent.

The disclosure concerning suitable and preferred embodiments stated above in connection with the explanation of the method for the preparation of a ready-to-use bone cement composition applies mutatis mutandum for this aspect of the invention.

The use according to this aspect of the invention is preferably for prophylactic or therapeutic treatment of a disorder related to supportive tissues involves fracture healing; insertion of prosthetic implants and implants of foreign materials in connection with fractures, skeletal defects, and osteotomy; non-instrumental non-invasive or invasive fusion surgery in connection with the spine or joints, preferably finger joints, vertebral joints and shoulder joints; prosthetic revision surgery; plastic surgery; reconstuction surgery; cosmetic surgery; sternotomics; trauma surgery; cancer surgery; oro-maxillar surgery; periodontitis; filling of maxillar, frontal, ethmoidal and spheroidal sinuses; creating room for an inflatable balloon or a metal expander; and infections or infestations in the musculoskeletal system, preferably osteomyelitis caused by bacteria.

**Description of the Preferred Embodiments**

The invention is now explained more in detail with reference to below Examples and the drawings explaining preferred embodiments of the invention.

MATERIALS AND METHODS

The ceramic bone substitute specimen contained 18.5 grams powder consisting of 59.6 wt% $\alpha$-CSH, 0.4% CSD and 40 wt% hydroxylapatite.

Vancomycin Hydrochloride from NordMedica

Iohexol solution comprising 180 mg I/ml was used.

Gillmore needles were used for characterization of setting time according to ASTM C266-04. The initial needle had a diameter of 2.12 $\pm$ 0.05 mm and a weight of 113 $\pm$ 0,5g. The final needle had a diameter of 1.06 $\pm$ 0.05 mm and a weight of 453.6 $\pm$ 0. 5g.

After mixing the paste was transferred into three moulds to form the test specimens. The initial and final setting time was taken as a mean value of the three tests.

The initial setting time is the time from the moment liquid is added to the bone substitute powder until the initial needle leaves no mark on the surface of the specimen. The final setting time is the time required for the paste to set so much that the final needle leaves no mark on the surface.

Qualitative and Quantitative phase analysis was performed using the Rietveld Method using a STOE θ/θ Diffractometer(X-ray Diffraction (XRD).

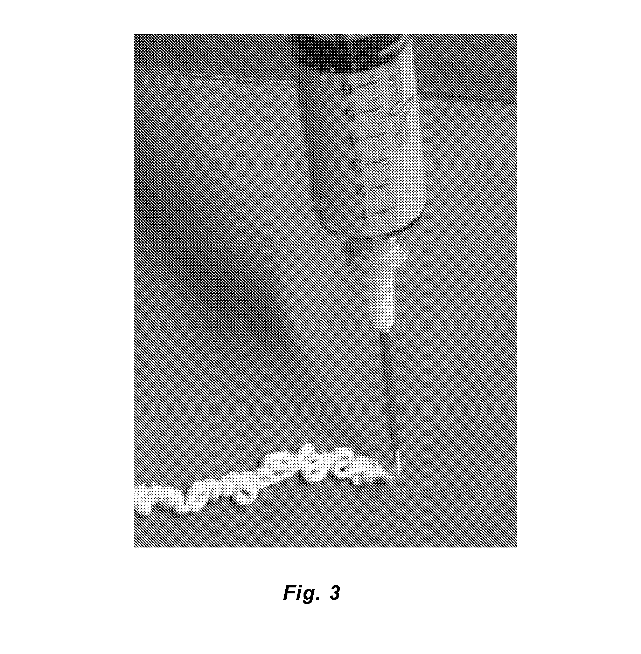

Injection test was performed using a 10 ml syringe with a 16 gauge needle by extruding out an approximately 5-10 cm string manually as shown in Figure 3 each minute until the time where no bone cement could be extruded.

## Example 1 and reference Examples 1 and 2

Preparation of a ceramic bone substitute paste from 18.5 gramspowder and 8 ml iohexol solution and 1 gram Vancomycin Hydrochloride from NordMedica (5.4 wt% vancomycin of powder weight). In Examples 1 and reference Example 1 two groups of materials having the same composition, i.e. 1 gram vancomycin, 18.5 grams specimen of ceramic bone substitute powder and 8 mℓ iohexol solution, were prepared, and the only difference between Example 1 and reference Example 1 was the method of mixing the materials.

In reference Example 2 a corresponding material without vancomycin was prepared. This material corresponds to a state of the art cement paste without antibiotic.

## Reference Example 1

Preparation of a ceramic bone substitute paste

1 gram vancomycin powder was first dissolved in 8 mℓ iohexol solution and then the resulting antibiotic-containing liquid was mixed with 18.5 grams powder for 30 s using a mixer of the kind disclosed in WO 2005/122971. The paste was used to prepare samples for characterization according to ASTM C266-04 and for XRD measurements.

## Example 1

Preparation of a ceramic bone substitute paste according to the invention

18.5 grams ceramic bone substitute powder and 8 mℓ iohexol solution were first mixed for 30 s using the mixer stated above in the absence of the vancomycin and thereafter transferred to a bowl. At 5 min 1 gram Vancomycin powder without lumps was added to the bowl and thereafter mixed manually with the pre-mixed paste with minimal extra energy using a spatula. The paste was immediately used to prepare samples for setting characterization according to ASTM C266-04 and for XRD measurements. Injection test through a 16 gauge needle was performed.

## Reference Example 2

Preparation of a ceramic bone substitute paste without vancomycin 18.5 grams powder and 8 mℓ iohexol solution were mixed for 30 s using the above mixer. The paste was used to prepare samples for setting characterization according to ASTM C266-04 and for XRD measurements. Injection through a 16 gauge needle was performed.

## Results

The results are summarized in the below Table 1.

The setting time measurements showed that the vancomycin-containing pastes prepared by dissolution of the Vancomycin powder in the liquid prior to the mixing with the hydraulic cement powder in reference Example 1 did not reach the initial setting time within the first 4.5 hours. The material finally became hard, but this is assumed to be due to drying.

In contrast to this, the vancomycin-containing paste prepared according to Example 1 gave much shorter setting times. By using this method, the initial setting time occurred in about 12 minutes and the final setting time in about 17 minutes. These setting times were quite similar to the ones obtained for the sample without vancomycin prepared according to Reference Example 2 (intial setting time about 8 minutes and final setting time at about 15 minutes).

Table 1: Results from setting time measurements and XRD analyses

| Example | | Reference Example 2 without medicament (reference) | Reference Example 1 (initial addition) | Example 1 (delayed addition |
|---|---|---|---|---|
| Setting time | Initial setting time | 8.2 ± 0.9 min | > 4,5 h | 12.1± 2.7 min |
| | Final setting time | 14.9 ± 1.0 min | > 4,5 h | 17.3 ±3.7 min |
| Phases in dry sample | Amount of CSD | 56.2 ± 1.2 wt% | 38.3 ± 1.0 wt% | 58.5 ± 1.3 wt% |
| | Remaining amount of CSH | 3.3 ± 0.6 wt% | 20.2 ± 0.9 wt% | 3.1 ± 0.4 wt% |
| Injection time through 16 gauge needle | | Possible until 6 min. | > 1 hour | Possible until 11 min. |

Minimum one sample was tested in each Example

In figure 2 is shown to what extent the CSH was converted to CSD in the three samples. The figures stated are the weight percentage of the CSD of the total amount of calcium sulfate

The XRD results stated in table 1 and figure 2 shows that the CSH in the sample prepared in reference Example 1 was only hydrated to about 2/3. This supports the assumption that the hardening of the cement in this case is partly due to drying of the mix and not completely due to setting. For the reference sample of Reference Example 2 and the sample prepared in Example 1, the XRD results showed that an almost complete CSH conversion to CSD.

**Discussion**

The results obtained using the method according to the invention indicates that it is generally applicable for the admixing of a wide range of different additives such as bioactive agents to calcium sulfate based compositions in which a hardening of calcium sulfate hemihydrate to calcium sulfate dihydrate is to take place.

Although it is possible to promote the dissolution of CSH and speed up the setting process, for example by adding accelerators such as NaCl it is, however, in many applications, important not to change the chemical composition of the material and addition of other chemicals should thus be avoided.

The present invention shows a drastic effect on the final properties of the material is obtained.

It was found that an important factor for affecting the setting times in the method of the invention is the mixing of the cement with the additive. It was found that it is important that this mixing is a short-duration mixing using a minimum of energy. The more energy that is used, the less viscous will the paste be and the advantage of using the method of the invention may be lost. A slow gentle manual mixing using a broad spatula is preferred.

When the bone cement paste is to be injected directly into a void it is very important to be able to control how the antibiotic addition is affecting the hardening properties of the paste. This is especially pertinent in environments having a high blood flow as a very slow setting of the paste and will increase the risk for leakage of the material. By using the method of the invention it was possible to shorten the setting times from > 4.5 h down to approximately 12 min (IST: 12.1 ± 2.7 min) when adding vancomycin to a bone cement paste. The method also gave a complete hydration of CSH to CSD.

**Summary Listing of References**

S. Gitelis and G.T. Brebach, Journal of Orthopaedic Surgery 2002 10(1): 53-60

N.B. Sing, B. M. (2007), Progress in Crystal Growth and Characterization of Materials 53, pp 57-77

WO 2004/078223 (BONESUPPORT AB)

K.C. Richelsoph, D.D. Webb and W.O. Haggard (2007) CLINICAL ORTHOPAEDICS AND RELATED RESEARCH, Number 461, pp 68-73

WO 01/76649 (BONESUPPORT AB)

WO 03/053488 (BONESUPPORT)

WO 2005/122971 (BONESUPPORT)

**Claims**

1. A method for the preparation of injectable ready-to-use bone cement paste compositions by mixing a dry inorganic bone cement powder comprising a particulate calcium sulfate hemihydrate capable of hardening *in vivo* by hydration of the calcium sulfate hemihydrate forming calcium sulfate dihydrate, an aqueous liquid and an additive, said method comprising

    a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
    b) mixing the bone cement powder with the aqueous liquid for a period of time
    c) allowing the calcium sulfate hemihydrate to dissolve at least partially and the formation of calcium sulfate dihydrate crystallization nuclei, and
    d) admixing the additive.

2. The method according to claim 1, wherein the calcium phosphate component is hydroxylapatite or tricalcium phosphate.

3. The method according to claim 1, wherein the additive is in the form of a bioactive agent.

4. The method according to any of claim 3, wherein the bioactive agent is an antibiotic agent.

5. An injectable paste composition comprising a particulate calcium sulfate hemihydrate capable of hardening *in vivo* by hydration of the calcium sulfate hemihydrate forming calcium sulfate dihydrate, an aqueous liquid and a bioactive agent, prepared by

    a) providing a bone cement powder comprising calcium sulfate hemihydrate, an accelerator for the hardening of the calcium sulfate hemihydrate by hydration, said accelerator being selected from the group consisting of saline and calcium sulfate dihydrate, and a powdered calcium phosphate component
    b) mixing the bone cement powder with the aqueous liquid for a period of time
    c) allowing the calcium sulfate hemihydrate to dissolve at least partially and the formation of calcium sulfate dihydrate crystallization nuclei, and

d) admixing the bioactive agent,

for use as a medicament for prophylactic or therapeutic treatment of a disorder related to supportive tissues in a human or non-human animal subject, which method comprises local administration to said subject, preferably by injection, of said composition comprising a prophylactic or therapeutic amount of said at least one bioactive agent, which is released from said composition, optionally while systemically and/or concomitantly administrating a prophylactic or therapeutic amount of at least one bioactive agent.

6. The paste according to claim 5, wherein the calcium phosphate component is hydroxylapatite or tricalcium phosphate.

7. The paste according to any of claims 5 or 6, wherein the bioactive agent is an antibiotic agent.

EP 2 353 619 A1

Fig. 1

Fig. 2

*Fig. 3*

Europäisches
Patentamt

European
Patent Office

Office européen
des brevets

# EUROPEAN SEARCH REPORT

Application Number

EP 10 15 3044

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/05861 A1 (BONE SUPPORT AB [SE]; LIDGREN LARS [SE]) 24 January 2002 (2002-01-24) * page 1, lines 7-11 * * page 7, line 25 - page 8, line 25 * * page 12, line 27 - page 13, line 26 * * claims 1,2,8,13-16 * | 5-7 | INV. A61L24/00 A61L24/02 A61L27/02 A61L27/12 A61L27/54 |
| X | US 6 251 139 B1 (LIN CHIH-I [US] ET AL) 26 June 2001 (2001-06-26) * column 1, lines 5-9 * * column 2, lines 4-25 * * examples 21-32 * | 1-7 | |
| A,D | GITELIS STEVEN ET AL: "The treatment of chronic osteomyelitis with a biodegradable antibiotic-impregnated implant." JOURNAL OF ORTHOPAEDIC SURGERY (HONG KONG) JUN 2002 LNKD- PUBMED:12401922, vol. 10, no. 1, June 2002 (2002-06), pages 53-60, XP002590798 ISSN: 1022-5536 * abstract * * page 54, column 2, paragraph 2 * | 1-7 | |
| A | WO 2005/099783 A1 (CORIPHARM MEDIZINPRODUKTE GMBH [DE]; KAPS HANS-PETER [DE]; SATTIG CHRI) 27 October 2005 (2005-10-27) * page 2, paragraph 3 * * page 7, paragraph 2 - page 8, paragraph 1 * * claims 1,2 * | 1-7 | TECHNICAL FIELDS SEARCHED (IPC) A61L |
| A | WO 01/34216 A1 (CORIPHARM MEDIZINPRODUKTE GMBH [DE]; WAHLIG HELMUT [DE]; DINGELDEIN EL) 17 May 2001 (2001-05-17) * page 1, lines 4-7 * * claims 1-3 * | 1-7 | |

The present search report has been drawn up for all claims

| Place of search | Date of completion of the search | Examiner |
|---|---|---|
| The Hague | 12 July 2010 | Cadamuro, Sergio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 10 15 3044

This annex lists the patent family members relating to the patent documents cited in  the above-mentioned European search report.
The members are as contained in the European Patent Office EDP file on
The European Patent Office is in no way liable for these particulars which are merely given for the purpose of information.

12-07-2010

| Patent document cited in search report | | Publication date | Patent family member(s) | | Publication date |
|---|---|---|---|---|---|
| WO 0205861 | A1 | 24-01-2002 | AT | 313344 T | 15-01-2006 |
| | | | AU | 7120901 A | 30-01-2002 |
| | | | AU | 2001271209 B2 | 15-12-2005 |
| | | | DE | 60116098 T2 | 17-08-2006 |
| | | | EP | 1301219 A1 | 16-04-2003 |
| | | | ES | 2252254 T3 | 16-05-2006 |
| | | | JP | 2004503332 T | 05-02-2004 |
| | | | SE | 517168 C2 | 23-04-2002 |
| | | | SE | 0002676 A | 18-01-2002 |
| | | | US | 2008286331 A1 | 20-11-2008 |
| | | | US | 2009018667 A1 | 15-01-2009 |
| | | | US | 2009192629 A1 | 30-07-2009 |
| | | | US | 2004048947 A1 | 11-03-2004 |
| US 6251139 | B1 | 26-06-2001 | CA | 2351105 A1 | 18-12-2002 |
| | | | EP | 1155704 A1 | 21-11-2001 |
| | | | JP | 2002331026 A | 19-11-2002 |
| WO 2005099783 | A1 | 27-10-2005 | DE | 102004016883 A1 | 27-10-2005 |
| | | | EP | 1732618 A1 | 20-12-2006 |
| | | | ES | 2314641 T3 | 16-03-2009 |
| WO 0134216 | A1 | 17-05-2001 | DE | 19953771 C1 | 13-06-2001 |
| | | | EP | 1227851 A1 | 07-08-2002 |
| | | | ES | 2199185 T3 | 16-02-2004 |
| | | | JP | 2003513711 T | 15-04-2003 |
| | | | US | 6689375 B1 | 10-02-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004078223 A, Lidgren
- WO 0176649 A
- WO 03053488 A
- WO 2005122971 A

**Non-patent literature cited in the description**

- **N. B. SINGH ; B. MIDDENDORF.** Calcium sulphate hemihydrate hydration leading to gypsum crystallization. *Progress in Crystal Growth and Characterization of Materials,* 2007, vol. 53, 57-77
- **STEVEN GITELIS ; GREGORY T. BREBACH.** The treatment of chronic osteomyelitis with a biodegradable antibiotic implant. *Journal of Orthopaedic Surgery,* 2002, vol. 10 (1), 53-60
- **RICHELSOPH et al.** Elution Behavior of Daptomycin-loaded Calcium Sulfate Pellets. *CLINICAL ORTHOPAEDICS AND RELATED RESEARCH,* 68-73
- **S. GITELIS ; G.T. BREBACH.** *Journal of Orthopaedic Surgery,* 2002, vol. 10 (1), 53-60
- **N.B. SING ; B. M.** *Progress in Crystal Growth and Characterization of Materials,* 2007, vol. 53, 57-77
- **K.C. RICHELSOPH ; D.D. WEBB ; W.O. HAGGARD.** *CLINICAL ORTHOPAEDICS AND RELATED RESEARCH,* 2007, 68-73